# EUROPEAN PATENT SPECIFICATION

(11) **EP 2 686 021 B1**
(45) Date of publication and mention of the grant of the patent: **15.08.2018**
(21) Application number: 12758097.5
(22) Date of filing: 14.03.2012
(51) Int. Cl.: A61K 48/00, A61K 36/31, A61K 36/752, A61K 31/122, A61P 17/00

(54) **ORAL FORMULATIONS FOR PROMOTING CELLULAR PURIFICATION**
ORALE FORMULIERUNGEN ZUR FÖRDERUNG EINER ZELLREINIGUNG
FORMULATIONS ORALES POUR PROMOTION DE PURIFICATION CELLULAIRE

(30) Priority: 14.03.2011 US 201161452478 P
(43) Date of publication of application: 22.01.2014
(73) Proprietor: NSE Products, Inc., Provo, UT 84601 (US)
(72) Inventor: MASTALOUDIS, Angela, Holladay Utah 84117 (US); WOOD, Steve, Santaquin Utah 84655 (US); BARGER, Jaime Louis, Verona Wisconsin 53593 (US); WEINDRUCH, Richard, Madison Wisconsin 53703 (US); POLLA, Tomas Alberto, Madison Wisconsin 53705 (US); BARTLETT, Mark, Orem Utah 84097 (US); FERGUSON, Scott B., Highland Utah 84003 (US)
(74) Representative: Walker, Ross Thomson
(86) International application number: PCT/US2012/029136
(87) International publication number: WO 2012/125772

(56) References cited:
- WO-A1-2010/118789
- WO-A2-2009/036204
- WO-A2-2011/149981
- GB-A- 2 346 325
- US-A1- 2004 001 817
- US-A1- 2007 248 705
- US-A1- 2008 305 096
- US-A1- 2009 069 417
- Albert Kompek: "Mediterrane sekundäre Pflanzenstoffe Resveratrol, Olivenpolyphenole und Lycopin in der Prävention altersbedingter Erkrankungen", ÖAZ Aktuell, vol. 4 15 December 2006 (2006-12-15), XP002727183, Retrieved from the Internet: URL:https://web.archive.org/web/2006121507 1351/http://www.oeaz.at/zeitung/3aktuell/2 006/04/haupt/haupt4_2006rotwein.html [retrieved on 2014-07-11]
- BALU M ET AL: "Age associated macromolecular damages in central nervous system of aged rats: Role of grape seed extract", MEDICINAL & AROMATIC PLANTS ABSTRACTS, SCIENTIFIC PUBLISHERS, NEW DELHI - INDIA, vol. 27, no. 1, 1 January 2005 (2005-01-01), XP018002046,
- ZHAO C R ET AL: "Nrf2-ARE signaling pathway and natural products for cancer chemoprevention", CANCER EPIDEMIOLOGY, ELSEVIER, AMSTERDAM, NL, vol. 34, no. 5, 1 October 2010 (2010-10-01), pages 523-533, XP027356195, ISSN: 1877-7821 [retrieved on 2010-09-28]
- SAW CONSTANCE LAY-LAY ET AL: "Anti-cancer and potential chemopreventive actions of ginseng by activating Nrf2 (NFE2L2) anti-oxidative stress/anti-inflammatory pathways", CHINESE MEDICINE, BIOMED CENTRAL LTD, LO, vol. 5, no. 1, 27 October 2010 (2010-10-27), page 37, XP021081707, ISSN: 1749-8546, DOI: 10.1186/1749-8546-5-37
- RAO, V. A. ET AL.: 'The antioxidant transcription factor Nrf2 negatively regulates autophagy and growth arrest induced by the anticancer redox agent mitoquinone' J. BIOL. CHEM. vol. 285, no. 45, 05 November 2010, pages 34447 - 34459, XP055122664

## Description

### RELATED APPLICATIONS

This application claims the benefit of U.S. Provisional Patent Application No. 61/452,478, which was filed on March 14, 2011.

### BACKGROUND

One way in which the aging process can manifest itself at the organismal level is changed ability to respond to oxidative stress and electrophilic insults, resulting in increased cellular damage. Such changes can in turn be a function of changes in various cell types that make up tissues and contribute to their function in organ systems. The activity, structure, and identity of a cell arises from its specific protein complement, as regulated by gene expression. As such, age-related changes in cellular structure and function likely find a basis in changes in genetic expression.

Through increasingly more sophisticated methods of measuring gene expression, it has become possible to identify genetic correlates of aging. For example, the use of whole genome transcriptional profiling, DNA microarrays, and quantitative PCR (qPCR), it is possible to identify transcriptional biomarkers of aging and to quantify the effects of aging on their expression. Interventions that retard or counteract these effects can therefore be beneficial in counteracting cellular, tissue, organ and organismal aging.

### SUMMARY

In the first aspect of the present invention there is provided an oral formulation comprising:
a plurality of agents, wherein the plurality of agents includes broccoli seed extract, red orange extract, and grape seed extract; and
wherein at least one of the plurality of agents modulates expression of Nrf2-associated genes upon ingestion of the oral formulation by a subject, wherein the Nrf2-associated genes include at least one gene encoding intrinsic antioxidants, and at least one gene encoding cellular detoxifiers, and wherein at least one of the plurality of agents attenuates inflammation.

In a further aspect of the present invention there is provided the use of the oral formulation of the present invention in therapy.

In another aspect of the present invention there is provided the non-therapeutic use of the oral formulation of the present invention for detoxification in cells of a subject.

In another aspect of the present invention there is provided a performance-enhancing formulation comprising a plurality of agents that enhance metabolic performance, and a detoxification formulation of the present invention for the use in a method of promoting health in a subject, the method comprising administering to the subject a performance-enhancing formulation and a detoxification formulation, wherein the detoxification formulation is to be administered to the subject within a 24-hour period of administering the performance enhancing formulation to the subject.

### DETAILED DESCRIPTION OF EXAMPLE EMBODIMENTS

In describing embodiments of the present invention, the following terminology will be used.

The singular forms "a," "an," and "the" include plural referents unless the context clearly dictates otherwise. Thus, for example, reference to "an agent" includes reference to one or more of such agents and "administering" includes one or more of such steps.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "50-250 milligrams should be interpreted to include not only the explicitly recited values of about 50 milligrams and 250 milligrams, but also include individual values and sub-ranges within the indicated range. Thus, included in this numerical range are individual values such as 60, 70, and 80 milligrams, and sub-ranges such as from 50-100 milligrams, from 100-200, and from 100-250 milligrams, etc. This same principle applies to ranges reciting only one numerical value and should apply regardless of the breadth of the range or the characteristics being described.

As used herein, the term "about" means that dimensions, sizes, formulations, parameters, shapes and other quantities and characteristics are not and need not be exact, but may be approximated and/or larger or smaller, as desired, reflecting tolerances, conversion factors, rounding off, measurement error and the like and other factors known to those of skill. Further, unless otherwise stated, the term "about" shall expressly include "exactly," consistent with the discussion above regarding ranges and numerical data.

As used herein, "up-regulation" and "down-regulation" refer respectively to increased or decreased expression of one or more genes and as a result the protein(s) encoded by those genes, e.g. in response to some signal, condition, or agent.

As used herein, "effective amount" refers to an amount of an ingredient which, when included in a composition, is sufficient to achieve an intended compositional or physiological effect. Thus, a "therapeutically effective amount" refers to a non-toxic, but sufficient amount of an active agent, to achieve therapeutic results in treating or preventing a condition for which the active agent is known to be effective. It is understood that various biological factors may affect the ability of a substance to perform its intended task. Therefore, an "effective amount" or a "therapeutically effective amount" may be dependent in some instances on such biological factors. Further, while the achievement of therapeutic effects may be measured by a physician or other qualified medical personnel using evaluations known in the art, it is recognized that individual variation and response to treatments may make the achievement of therapeutic effects a subjective decision. The determination of an effective amount is well within the ordinary skill in the art of pharmaceutical and nutritional sciences as well as medicine.

As used herein, "pharmaceutically or nutritionally acceptable carrier," and "carrier" may be used interchangeably, and refers to any inert and pharmaceutically or nutritionally acceptable material with which a bioactive agent or a nutritional agent may be combined to achieve a specific dosage formulation for delivery to a subject. As a general principle, carriers must not react with the bioactive agent in a manner which substantially degrades or otherwise adversely affects the bioactive agent.

As used herein, "attenuation" of a process includes results in which the process is slowed, halted, reversed, or prevented from increasing. In a particular example, attenuation of inflammation can be achieved by slowing or halting of pro-inflammatory processes and pathways, as well as by up-regulating anti-inflammatory processes and pathways.

As used herein, "Nrf2-associated genes" refers to genes (e.g. NFE2L2) that encode for Nuclear Factor (erythroid derived 2)-like 2 protein (referred to herein as "Nrf2") as well as genes in which expression can be modulated by the binding of Nrf2 to antioxidant response elements (AREs) associated with these genes.

As used herein, "excipient" refers to substantially inert substance, which may be combined with an active agent and a carrier to achieve a specific dosage formulation for delivery to a subject, or to provide a dosage form with specific performance properties. For example, excipients may include binders, lubricants, etc., but specifically exclude active agents and carriers.

As used herein, "subject" refers to a mammal that may benefit from the administration of a composition or method as recited herein. Most often, the subject will be a human.

As used herein, "administration," and "administering" refer to the manner in which an active agent, or composition containing such, is presented to a subject. Administration can be accomplished by various routes well-known in the art such as oral and non-oral methods.

As used herein, "oral administration" refers to a route of administration that can be achieved by swallowing, chewing, or sucking of an oral dosage form comprising the drug or nutritional formula. Examples of well-known oral dosage forms include tablets, capsules, caplets, powders, granulates, beverages, syrups, elixirs, confections, or other food items, etc.

The present technology includes a novel nutritional intervention to enhance cellular purification and oppose or attenuate the negative effects of aging. Oxidative injury, electrophilic damage and inflammation are intimately involved in the aging process and the development of age-related diseases. Conventional anti-aging strategies have typically focused solely on the delivery of exogenous antioxidants to combat the negative effects of aging. The present innovation reflects a new strategy of identifying natural compounds that can directly target intrinsic cytoprotective mechanisms including: 1) upregulation of genes involved in the detoxification of xenobiotics and xenobiotic metabolites, 2) upregulation of genes involved in the synthesis and regulation of intrinsic antioxidants and antioxidant enzymes and 3) modulation of genes involved in the attenuation of inflammation.

In particular, compounds that modulate genes in the key age-related pathways mediated by Nrf2 can be employed in formulations that promote cellular purification and enhance intrinsic antioxidant responses. Nrf2 is a transcription factor that positively regulates the basal and inducible expression of a large battery of genes encoding for cytoprotective factors including those that defend against electrophilic stressors and oxidative insults. Nrf2 activity has been observed upon exposure of cells to oxidative and electrophilic stress. The following are non-limiting examples of Nrf2-associated genes:
**NFE2L2** (Nuclear factor, erythroid derived 2, like 2). Codes for the Nrf2 transcriptional factor responsible for both inducible and constitutive expression of antioxidant response element (ARE)-regulated genes, including those coding for a number of antioxidant proteins and Phase II detoxifying enzymes that defend against electrophilic stressors and oxidative insults.
**GCLM and GCLC.** Glutamate-cysteine ligase, also known as gamma-glutamylcysteine synthetase, is the first rate limiting enzyme of glutathione (GSH) synthesis. The enzyme consists of two subunits, a heavy catalytic subunit (GCLC) and a light regulatory subunit (GCLM). Overexpression of GCLC or GCLM in fruit flies extends lifespan, without affecting the rate of oxygen consumption.
**GSR** encodes a member of the class-I pyridine nucleotide-disulfide oxidoreductase family, glutathione reductase (GSR). This is a central enzyme in cellular antioxidant defense, and reduces oxidized glutathione disulfide (GSSG) to the sulfhydryl form GSH.
**GSTA1** encodes an alpha class glutathione S-tranferase, which functions in the detoxification of electrophilic compounds, including carcinogens, therapeutic drugs, environmental toxins and products of oxidative stress, by conjugation with GSH. In addition to metabolizing bilirubin and certain anti-cancer drugs in the liver, alpha class glutathione S-tranferases exhibit glutathione peroxidase activity, thereby protecting the cells from reactive oxygen species and the products of peroxidation,
**GPX1** encodes for glutathione peroxidase, an intrinsic antioxidant enzyme responsible for the removal of the damaging reactive oxygen species hydrogen peroxide (H₂O₂) and synthetic organic peroxides, utilizing GSH as an electron donor.
**GPX4** encodes for phospholipid hydroperoxide glutathione peroxidase, an intrinsic antioxidant enzyme with the same activity as GPX1, but with the additional ability to remove the metabolic toxicants fatty acid hydroperoxides and cholesterol hydroperoxides.
**SOD1** encodes for the soluble form of copper-zinc-superoxide dismutase (CuZnSOD1), an intrinsic antioxidant enzyme involved in the catalytic removal of the reactive superoxide radical (O₂).
**HMOX1** encodes for heme oxygenase (HO-1), the inducible isoform of the first and rate-limiting enzyme of heme degradation. HO-1 has potent antioxidant and also anti-inflammatory functions. Induction of HO-1 protects against the cytotoxicity of oxidative stress and apoptotic cell death.
**NQO1** is a member of the NAD(P)H dehydrogenase (quinone) family and encodes a cytoplasmic 2-electron reductase. Altered expression of this protein has been observed in many tumors and is also associated with Alzheimer's disease (AD).
**SRXN1,** a key Nrf2-regulated gene, contributes to protection against oxidative injury in the lung. Disruption of Nrf2 signaling by genetic knockout in mice or RNAi in cells downregulated the expression of Srxl. *In silico* analysis of the 5'-promoter-flanking region of SRXN1 has identified multiple antioxidant-response elements (AREs) that are highly conserved. Reporter and chromatin-immunoprecipitation assays have demonstrated that ARE1 at -228 is critical for the Nrf2-regulated response. Attenuation of SRXN1 expression with RNAi potentiated the toxicity of H₂O₂, whereas overexpression of SRXN1 protected against H2O₂-mediated cell death in vitro,
**UGT1A6** encodes a UDP-glucuronosyltransferase, an enzyme of the glucuronidation pathway that transforms small lipophilic molecules, such as steroids, bilirubin, hormones, and drugs, into water-soluble, excretable metabolites. The enzyme encoded by this gene is active on phenolic and planar compounds.
**NOS2.** Nitric oxide is a reactive free radical which acts as a biologic mediator in several processes, including neurotransmission and antimicrobial and anti-tumoral activities. This gene encodes the inducible nitric oxide synthase (iNOS) which is highly expressed in liver.
**NOS3** encodes for endothelium-derived NOS (eNOS) which is responsible for the production of nitric oxide necessary for vasodilation; dysregulated in inflammatory conditions and in aging.
**PTGS2.** Prostaglandin-endoperoxide synthase (PTGS), also known as cyclooxygenase 2 (COX2), is the key enzyme in prostaglandin biosynthesis, and acts both as a dioxygenase and as a peroxidase. There are two isozymes of PTGS: a constitutive PTGS1 and an inducible PTGS2, which differ in their regulation of expression and tissue distribution. This gene encodes the inducible isozyme. It is regulated by specific stimulatory events, suggesting that it is responsible for the prostanoid biosynthesis involved in inflammation and mitogenesis.

In an embodiment of the present technology, an oral formulation comprises a plurality of agents that modulate expression of Nrf2-associated genes upon ingestion of the oral formulation by a subject. The genes modulated can be any of those encoding for products that act as antioxidants in cells or enzymes that mediate redox reactions, as well as proteins that are involved in removal or recycling waste products. In particular, the Nrf2-associated genes can include at least one gene encoding intrinsic antioxidants, and at least one gene encoding cellular detoxifiers. In another aspect, at least one of the plurality of agents attenuates inflammation. The effect on inflammation can involve modulating gene expression. For example, inflammation can be attenuated by down-regulating expression of gene products that contribute to inflammatory pathways (e.g. TNF-α), and/or by up-regulating expression of anti-inflammatory proteins (e.g. cytokine IL-10).

In still another aspect, at least one of the agents can stimulate inducible autophagy in such a way that enhances response to oxidative stress. The protein p62 is known to be crucial for the formation of ubiquitylated protein aggregates. P62 also interacts with Keap1, a component of a ubiquitin ligase complex that mediates proteasomal degradation of Nrf2. However, the inhibitory effect of Keap1 on Nrf2 is dependent on the redox status of Keap1 cysteines, such that Nrf2 ubiquitination and proteolysis are inhibited in oxidized conditions. Decreased autophagy results in accumulation of p62 in the cytoplasm and more binding of Keap1. Conversely, increased induction of autophagy can result in decreased availability of p62 for interaction with Keap1, which can preserve the ability of Keap1 to regulate Nrf2 oxidative response.

In accordance with the present technology, the formulation can include agents that are selected based on their action on particular Nrf2-associated genes or panels or pathways of such genes. As noted above, one such group of genes can include but is not limited to NFE2L2, GCLM, GCLC, GSR, GSTA1, GPX1, GPX4, HMOX1, NQO1, SRXN1, SQSTM1, SOD1, UGT1A6, NOS2, NOS3, and PTGS2. In one example, the formulation can include agents that modulate a plurality of Nrf2-associated genes, In a further aspect, the formulation can include a plurality of such agents selected so that the combined agents modulate at least some minimum number of Nrf2-associated genes. In accordance with the present technology, the agents in such a formulation can combine to modulate at least three such genes but in a more specific embodiment of modulating at least four to five of such genes, It is noted that this is but one example, so many combinations of agents can be selected to combine to modulate other numbers of genes.

Administration of the oral formulation can lessen the impact of aging on cytoprotective mechanisms. In an aspect, use of the formulation in an aged subject can oppose, attenuate, or reverse age-related effects on these mechanisms. In a particular aspect the oral formulation is effective in opposing, attenuating, or reversing age-related changes in the expression of genes whose transcription products are involved in cytoprotective mechanisms. In one aspect, administration of the oral formulation substantially reverses an about 1.1 fold to about 3.0 fold age-related downregulation of Nrf2, In another aspect, administration of the oral formulation opposes age-related downregulation of genes involved in glutathione synthesis. In an example, the oral formulation reverses an age-related decrease of 1.1 fold to 3.0 fold in expression of any of GCLC, GCLM, and GSR. In another example, the oral formulation is effective to substantially reverse an age-related decrease of from about 1.5 fold to about 6.0 fold in expression of GSTA1.

In another aspect, administration of the oral formulation reverses age-related upregulation of antioxidant and detoxification gene expression. In an example, the oral formulation is effective to substantially reverse an age-related increase of about 1.05 to about 4.0 fold in expression of any of Gpx1, Gpx4, and SRXN1. In another example, the oral formulation is effective to substantially reverse an age-related increase of about 1.05 to about 3.0 fold in expression of SOD1.

In another aspect, the oral formulation is effective to reverse age-related effects in expression that is associated with inflammation. In an example, the oral formulation is effective to substantially reverse an age-related increase of about 1.05 to about 3.0 fold in expression of either of NOS2 and NOS3.

In another aspect of the present technology, the oral formulation is effective to substantially reverse age-related changes in DNA stability. In an example, the formulation reverses age-related downregulation of pathways for DNA repair. In another example, the formulation can exert other protective effects such as upregulating pathways for telomere maintenance and organization. In another aspect, the oral formulation is effective to substantially reverse age-related changes in autophagy. In another aspect, the oral formulation is effective to substantially reverse age-related changes in inflammatory responses.

The plurality of agents in the formulation comprises natural compounds such as nutrients and plant extracts that, when ingested, modulate the expression of Nrf2-associated genes. Such compounds include broccoli seed extract, alpha lipoic acid, red orange extract, grape seed extract, whole grape extract, ginseng, olive leaf extract, olive fruit extract, coenzyme Q₁₀, pomegranate extract, curcumin, EGCG, lutein, lycopene, zeaxanthin, resveratrol, Schizandra berry extract, tart cherry extract, rosemary extract, and *Cordyceps sinensis.*

As discussed above, different combinations of agents can be included to provide different effective modulation profiles. According to the present invention, broccoli seed extract, red orange extract, and grape seed extract are combined in an oral formulation. In a more specific embodiment, the formulation can include from 20 to 30 wt% broccoli seed extract, from 25 wt% to 35 wt% red orange extract, and from 45 to 55 wt% grape seed extract. In another disclosure the formulation comprises olive leaf extract, olive fruit extract, red orange extract, grape seed extract, and coenzyme Q₁₀. In such a more specific disclosure the formulation comprises from 25 to 35 wt% olive leaf extract, from 5 wt% to 15 wt% olive fruit extract, from 15 wt% to 25 wt% red orange extract, from 25 to 35 wt% grape seed extract, and from 5 to 15 wt% coenzyme Q₁₀.

Consistency in the results of using the formulation can be enhanced by standardizing the ingredients according to certain active constituents. For example, in an aspect of the above embodiments, grape seed extract can be standardized to contain from about 50% to about 99% polyphenols. In a specific example, the grape seed extract contains about 95% polyphenols. In another aspect, the red orange extract can be standardized to contain from about 2.5%) to about 25%) polyphenols. In a specific example, the red orange extract contains about 15% polyphenols. In another aspect, the broccoli seed extract used is standardized to contain from about 1% to about 20%. sulphoraphane. In one specific example, the broccoli seed extract contains about 13% sulphoraphane.

The oral formulation can be prepared in any delivery or dosage form suited for oral administration. For example the active agents in the formulation can be combined with a liquid carrier and then concentrated or diluted to prepare a liquid form. Alternatively, the active agents can be dried, processed, and combined with appropriate materials such as carriers, fillers, tabletting agents, plasticizers, and the like for preparation of a solid dosage form. In some aspects, the oral formulation may consist essentially of a dried and powdered form of the active agents, or an extract from a natural source containing the active agent, which is packaged and presented for suitable oral administration. Solid and liquid dosage forms known in the food and pharmaceutical arts are contemplated to be used, such as capsules, tablets, powders, beverages, wafers, confectionaries, chewables, gels, pastes, elixirs, syrups, drops, lozenges, and the like, In a particular embodiment, the oral formulation is processed into a powder that may optionally include sweeteners and flavors and is dissolvable in water or other liquid to create a beverage. In another particular embodiment, the oral formulation is processed and placed in a capsule, such as a gelatin capsule.

The oral formulation can further include one or more excipients as called for to prepare a delivery form. A variety of excipients commonly known in the pharmaceutical, nutritional supplement and food industry for making various dosage forms may be used. These include, for example, liquid carriers, solvents, fillers, binders, lubricants, glidants, flavorings, and colorings. In a particular embodiment, the oral formulation includes one or more of food grade gum, anti-caking agents, lecithin, microcrystalline cellulose, silica gel, flavoring, and sweetener. Food grade gums include xanthar gum and guar gum. Anti-caking agents include without limitation silicon dioxide, stearic acid, tricalcium phosphate, calcium silicate, sodium aluminosilicate, magnesium carbonate, talc, bentonite, sodium ferrocyanide, potassium ferrocyanide, and bone phosphate.

In accordance with the present technology, a method for promoting cellular purification, or cleansing can comprise administering to the subject an oral formulation comprising a plurality of agents that modulate expression of Nrf2-associated genes. The Nrf2-associated genes can include at least one gene encoding intrinsic antioxidants, and at least one gene encoding cellular detoxifiers. In a further aspect, at least one of the plurality of agents attenuates inflammation. In another aspect at least one of the plurality of agents stimulates autophagy in tissues of the subject.

The oral formulation can be formulated to provide an effective amount of the active agents in accordance with a particular dosage regimen. The oral formulations herein can provide each of the active agents according to a desired daily dose. In a specific embodiment, administering the oral formulation provides the subject with a daily dosage of 125 mg red orange extract, 210 mg grape seed extract, and 115 mg broccoli seed extract.

In another aspect, the oral formulation can be administered to a subject so as to deliver a desired amount of active agent on a per body weight basis. Administration can be configured based on the species of subject (e.g. a mammalian subject, or more specifically a human subject), as well as other factors such as sex, age, medical condition, and the like. In a particular embodiment, an effective amount of the oral formulation delivers to the subject a daily dose per kg of body weight comprising from about 0.15 to about 18 mg red orange extract, from about 0.3 to about 30 mg grape seed extract, and from about 0.15 to about 16.5 mg broccoli seed extract.

Normal cellular activity during a typical day can eventually result in oxidative stress and accumulation of metabolic waste products, which are increased by strenuous activity or other elevated stressors, a problem which is exacerbated with increasing age. Therefore, there can be a particular need for cellular detoxification and antioxidant response after a period of prolonged activity, e.g. at the end of the day. Accordingly, one indication for use of the formulations described herein is administration at night. However, the time of administration can be selected based on the activity cycle of the subject. For example, a subject who engages in prolonged nocturnal activity (e.g. nighttime shift work) can benefit from taking the formulation in the morning after the active period is completed. In a specific aspect, therefore, the formulation can be administered to the subject at whatever time the subject retires to bed.

It is further contemplated that the formulations and uses discussed herein can be employed in conjunction with other treatments. For example, the formulations discussed herein can be used in conjunction with a performance-enhancing formulation such as described in U.S. Patent Application No. 13/115,027. In one example, the performance-enhancing formulation comprising agents that enhance metabolic performance can be taken in the morning, and a detoxification formulation according to the present technology can be taken at night. The increased activity facilitated by the performance-enhancing formulation can result in metabolic effects such as increased oxidative stress and increased metabolic waste, and therefore a greater need for the detoxification and recovery provided by the present formulation. As such, the concomitant administration of the two formulations within a single 24-hour period may be of substantial benefit in improving the health and well being of a subject as an overall system or program, as compared to the use of just one of the formulations alone. In accordance with another embodiment, a performance-enhancing formulation as described above and a detoxification formulation according to the present technology can be included in a kit. The kit can further include user instructions guiding a user to administer each formulation according to a particular timetable, for example within a 24-hour period of each other.

The aspects of the present invention are illustrated further by the following exemplary embodiments. These examples should not be considered as limitations of the disclosure, but are merely in place to instruct those skilled in the art in practicing the invention. It will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

### EXAMPLES (including reference ingredients)

### Example 1 - Testing effects of ingredients on Nrf2-associated gene expression in liver and lungs of young mice

### Feeding protocol:

C57BL/6J mice were obtained at 6 weeks of age and individually housed in shoebox cages and provided with 24 grams (∼84 kcal) of AIN-93^{M} diet per week (7 grams on Monday and Wednesday and 10 grams on Friday). Starting at 8 weeks of age and continuing until 22 weeks of age, mice were either 1) maintained on the AIN93^{M} diet (Young Controls, YC); 2) fed a Calorie Restricted (CR) diet providing 63 kcal/week of a modified AIN93^{M} formulation; or 3) were assigned to an AIN93^{M} diet supplemented with one of several plant extracts, vitamins or phytochemicals; the amount of each ingredient per kilogram diet varied depending on the experimental ingredient studied. At 22 weeks of age, tissues were collected from mice, flash-frozen in liquid nitrogen and stored at - 80°C for later analysis.

### Ingredients tested:

Quercetin (from Fava d'Anta), Tart cherry (*Prunus cerasus* L. (Rosaceae) cv. Balaton), Carnosol (Rosemary (*Rosmarinus officinalis Linn.,* carnosic acid), Broccoli seed extract (13% Sulphoraphane glucosinolate), Alpha lipoic acid, PowerGrape™ (whole grape extract), Grape seed extract (GSE), Olive leaf and olive water extracts (hydroxytyrosol/Oleuropein), *Schizandra chinensis, Cordyceps sinensis,* pomegranate extract, Panax ginseng and CoQ₁₀/Ubiquinol.

### Screening:

To determine if CR or an ingredient positively influenced the Nrf2 pathway to regulate expression of xenobiotic metabolism genes and oxidative stress genes and/or positively influenced genes responsible for regulation of inflammation, we performed quantitative real-time PCR (RT-qPCR) analysis on RNA isolated from entire livers and lungs from all groups of mice. Briefly, the magnitude of change was determined for each gene, comparing the young control (YC) group vs. the caloric or energy restriction (CR) group and YC vs. Treated mice. Two-tailed *t*-tests (assuming equal variance) were used to determine if the change in expression for individual genes was statistically significant. The magnitude of the change in expression is reported as "fold change" values which are log₂-adjusted to fit normality assumptions for statistical analyses.

A panel of 10 genes representative of the Nrf2 and inflammatory pathways was selected for screening the ingredients: GCLM, GCLC, GSR, GSTA1, HMOX1, NQO1, SRXN1, UGT1A6, NOS2, and PTGS2,

### Results

### Liver tissue:

Robust gene expression changes were seen in the liver in response to CR and to various nutrients. Ingredients with greatest positive impact included broccoli seed extract (sulphoraphane), blood orange extract, alpha lipoic acid and olive extracts. Most robust changes were in the Nrf2/ARE/detoxification related genes. Anti-inflammatory genes remained relatively unaffected with one exception. COX2/Ptgs2 was downregulated 2 fold with CR (as would be predicted), however it was upregulated more than 2 fold by Schisandra, suggesting the possibility of pro-inflammatory effects of this plant material.

### Lung tissue:

Very few changes in gene expression were observed in the lung regardless of the intervention, CR or nutrient. Of the changes observed, greatest benefit was down-regulation of the anti-inflammatory gene iNOS/Nos2, Modest downregulation of iNOS was observed with CR, CoQ₁₀ and quercetin.

### Example 2 - Comparing effects of ingredients and CR on Nrf2-associated gene expression in gastrocnemius of young and old mice

### Feeding protocol:

B6C3F1 mice were obtained and housed as in Example 1, For the young control (YC) group, starting at 8 weeks of age and continuing until 22 weeks of age, mice were maintained on the AIN93^{M} diet; For the old animal groups, starting at 14 months of age and continuing until 30 months of age, mice were either 1) maintained on the AIN93^{M} diet (old controls, OC); 2) fed a calorie or energy restricted (CR) diet providing 63 kcal/week of a modified AIN93^{M} formulation; or 3) were assigned to an AIN93^{M} diet supplemented with one of several plant extracts, vitamins or phytochemicals; the amount of each ingredient per kilogram diet varied depending on the experimental ingredient studied. At the end of the feeding period, tissues were collected from the gastrocnemius muscles of the mice, flash-frozen in liquid nitrogen and stored at -80°C for later analysis,

### Screening

To determine if CR or an ingredient opposed age-related declines in the Nrf2 pathway to regulate expression of xenobiotic metabolism genes and oxidative stress genes, we performed quantitative real-time PCR (RT-qPCR) analysis on RNA isolated from gastrocnemius muscle from all groups of mice. The magnitude of change was determined for each gene (YC vs. OC and OC vs Old treated mice). Two-tailed *t*-tests (assuming equal variance) were used to determine if the change in expression for individual genes was statistically significant. The magnitude of the change in expression is reported as "fold change" values which are log₂-adjusted to fit normality assumptions for statistical analyses.

A panel of 5 genes representative of the Nrf2 pathway was selected for screening the ingredients: GCLM, GCLC, GSR, GSTA1, and NQO1. Ingredients screened in the muscle included: alpha lipoic acid, CoQ₁₀, Pomegranate, Resveratrol and others.

### Results

Alpha lipoic acid upregulated GCLC (glutathione synthesis related gene) and GSR (maintains GSH in reduced form, indicator of oxidative stress status). Furthermore, alpha lipoic acid opposed age-related decreases in the expression of GCLC and GSR. CoQ₁₀ upregulated GCLC and opposed age related decreases in the expression of GCLC. Pomegranate extract upregulated GCLM (glutathione synthesis related gene) and opposed age-related decreases in the expression of GCLM.

### Example 3 - Testing effects of ingredients on Nrf2-associated gene expression in young and middle-aged mice

### Feeding protocol:

CBA/J mice are fed one of four mixtures of compounds that were tested singly under approved VA Animal Care Protocols. These mixtures are fed to middle-aged mice (-15 months of age). All mice are individually housed and fed defined AIN93^{M} diets in calorie-controlled amounts as in Example 1. At the end of the experiments, tissues are collected from mice to determine if the mixtures had the ability to modify the pathways of interest and/or slow the aging process.

These studies are performed on CBA/J mice starting during adolescence (∼2 months of age) or starting in middle age (∼15 months of age) and extending 3-5 months (2-5 or 18-20 months of age). The following groups are studied:
1. Young Controls (YC) (n=8 mice) fed an AIN93^{M} diet alone are used to establish a baseline for youthful gene expression;
2. Middle-Aged Controls (MAC) (n=8 mice) fed an AIN93^{M} diet alone are used to establish a baseline for gene expression with age in the absence of treatment;
3. Middle-Aged Mice fed an AIN93^{M} diet fortified with one of four mixtures of dietary compounds (n=64 mice for 4 all diets).
   a. Treatment 1. red orange extract, grape seed extract and broccoli seed extract
   b. Treatment 2. *Cozdyceps sinensis*, pomegranate extract and *Panax ginseng* extract (reference mixture)
   c. Treatment 3 red orange extract, grape seed extract, coenzyme Q₁₀, olive leaf extract and olive fruit extract (reference mixture)
   d. Treatment 4. red orange extract, grape seed extract, broccoli seed extract, *Cordyceps sinensis,* pomegranate extract and *Panax ginseng* extract

Gene expression profiling is used to identify individual genes and functional classes of genes that are changed with treatment. Liver, adipose, heart, brain, lung and gastrocnemius muscle are examined. Detailed experimental methods for sample preparation and microarray analysis are published elsewhere (http://dx.doi.org/10.1073/pnas.232308999). One exception is that for this experiment, the Affymetrix Mouse 1.0 Gene ST array is utilized, which allows for the detection of 20,696 unique genes.

To identify changes in gene expression that occur with age, the average value of the Middle-Aged Controls samples are compared with the average values of the Young Controls. To identify changes in gene expression that occur with treatment, the average value of the Treatment samples are compared with the average values of the Middle-Aged Controls. Two-tailed *t*-tests (assuming equal variance) are used to determine if the change in expression for individual genes is statistically significant. The magnitude of the changes in expression are reported as "fold change" values which are log₂-adjusted to fit normality assumptions for statistical analyses.

To identify functional classes or pathways of genes changed with Treatment, Parametric Analysis of Gene set Enrichment (PAGE) is applied as described previously (http//dx.doi.org/10.1186/1471-2105-6-6-144). Annotations from the Gene Ontology (GO) consortium are used to link individual genes with their function (http://www.geneontology.org). Annotations from "Level 3" or greater are included and only those GO terms that are represented by more than 10 but less than 1000 genes are considered. The PAGE technique also calculates a z-score for each GO term, with positive values indicating that a GO term is upregulated with treatment and negative values indicating downregulation of a GO term by treatment.

Microarray findings are confirmed by quantitative real-time PCR (RT-qPCR) analysis on RNA isolated from tissues from all groups of mice using a representative subset of genes. The magnitude of change is determined for each gene (YC vs. MAC; MAC vs. treated mice). Two-tailed *t*-tests (assuming equal variance) are used to determine if the change in expression for individual genes is statistically significant. The magnitude of the change in expression is reported as "fold change" values which are log₂-adjusted to fit normality assumptions for statistical analyses.

### Results

Supplementation of middle-aged mice supplemented with mixtures of nutritional compounds are seen to oppose or attenuate age-related changes in gene expression pathways related to Phase II detoxification pathway and other cytoprotective pathways. They also oppose or attenuate age-related changes in genes related to the control of autophagic and inflammatory regulation. Finally, they oppose age-related declines in the expression of genes responsible for antioxidant protection mechanisms. The supplement blends have greater effects in opposing age-related changes in gene expression than the effects of the individual ingredients fed alone. The blends oppose: 1) downregulation of genes involved in the detoxification of xenobiotic and xenobiotic metabolites, 2) downregulation of genes involved in the synthesis and regulation of intrinsic antioxidants and antioxidant enzymes, 3) downregulation of genes involved in autophagic control and 4) age-related modulation of genes involved in the regulation of inflammation.

### Example 4 - Formulations

Based on the above results, effective ingredients were combined in two formulations as shown below:

**Table 1**

| **Ingredient** | **Label Amt. per day (mg)** | **Activity Factor** | **Min. Overage** | **Minimum Ingredient per Dosage Unit (mg)** |
|---|---|---|---|---|
| Broccoli seed extract (sulphoraphane/glucosinolate) | 15 | 0.130 | 1.050 | 121.154 |
| Red Orange Extract (15% polyphenols (PPs)) | 25.0 | 0.200 | 1.050 | 131.250 |
| Grape Seed Extract (95% PP) | 200.0 | 0.950 | 1.050 | 221.053 |

**Table 2 (reference mixture)**

| **Ingredient** | **Label Amt. per day (mg)** | **Activity Factor** | **Min. Overage** | **Minimum Ingredient per Dosage Unit (mg)** |
|---|---|---|---|---|
| Olive Leaf Extract (Oleuropein 20%) | 40.0 | 0.200 | 1.050 | 210.000 |
| Olive Fruit Extract (6% Hydroxytyrosol) | 4.0 | 0.060 | 1.050 | 70.000 |
| Red Orange Complex (15% PPs) | 25.0 | 0.200 | 1.050 | 131.250 |
| Grape Seed Extract (95% PPs) | 200.0 | 0.950 | 1.050 | 221.053 |
| Coenzyme Q₁₀ (ubiquinone 20%) | 15.0 | 0.200 | 1.050 | 78.750 |

## Claims

1. An oral formulation comprising:
a plurality of agents, wherein the plurality of agents includes broccoli seed extract, red orange extract, and grape seed extract; and
wherein at least one of the plurality of agents modulates expression of Nrf2-associated genes upon ingestion of the oral formulation by a subject, wherein the Nrf2-associated genes include at least one gene encoding intrinsic antioxidants, and at least one gene encoding cellular detoxifiers, and wherein at least one of the plurality of agents attenuates inflammation.

2. The oral formulation of claim 1, wherein the Nrf2-associated genes are selected from the group consisting of NFE2L2, GCLM, GCLC, GSR, GSTA1, GPX1, GPX4, HMOX1, NQO1, SRXN1, SQSTM1, SOD1, UGT1A6, NOS2, NOS3, and PTGS2.

3. The oral formulation of claim 2, wherein at least one of the plurality of agents substantially reverses an age-related change in expression of at least one of the Nrf2-associated genes.

4. The oral formulation of claim 1, wherein the plurality of agents combine to modulate-expression of at least five Nrf2-associated genes.

5. The oral formulation of claim 1, wherein at least one of the plurality of agents upregulates a gene encoding intrinsic antioxidants, or a gene encoding cellular detoxifiers, or stimulates autophagy in a tissue of a subject.

6. The oral formulation of claim 1, wherein the plurality of agents additionally includes at least one of alpha lipoic acid, whole grape extract, olive leaf extract, olive fruit extract, coenzyme Q₁₀, pomegranate extract, curcumin, EGCG, lutein, lycopene, zeaxanthin, resveratrol, Schizandra berry extract, tart cherry, ginseng, rosemary extract, and *Cordyceps sinensis.*

7. The oral formulation of claim 1, wherein the formulation comprises from 20 to 30 wt% broccoli seed extract, from 25 wt% to 35 wt% red orange extract, and from 45 to 55 wt% grape seed extract.

8. The oral formulation of claim 1, present in a dosage form selected from the group consisting of: capsule, tablet, powder, beverage, wafer, confectionary, chewable, gel, paste, elixir, syrup, drops, and lozenge.

9. The oral formulation of claim 1, further comprising at least one excipient selected from the group consisting of microcrystalline cellulose, silicon dioxide, stearic acid, a food-grade gum, lecithin, and an anti-caking agent.

10. The oral formulation of any one of the preceding claims for use in a therapy.

11. The oral formulation of any one of the preceding claims for use in promoting therapeutic detoxification in cells of a subject.

12. Use of the oral formulation of any one of claims 1 to 9 for detoxification in cells of a subject, wherein the use is not for the purposes of therapy carried out on the human or animal body.

13. The oral formulation for the use of claim 10 or 11, wherein the plurality of agents combine to modulate expression of at least five Nrf2-associated genes.

14. The oral formulation for the use of claim 10 or 11, wherein at least one of the plurality of agents stimulates autophagy in a tissue of the subject.

15. The oral formulation for the use of claim 10 or 11, wherein administering provides the subject with:
a daily dosage of 125 mg red orange extract, 210 mg grape seed extract, and 115 mg broccoli seed extract; or
a dosage per kg of body weight of from 0.15 mg to 18 mg red orange extract, from 0.3 mg to 30 mg grape seed extract, and from 0.15 mg to 16.5 mg broccoli seed extract.

16. A performance-enhancing formulation comprising a plurality of agents that enhance metabolic performance, and a detoxification formulation as recited in any of claims 1-9 for the use in a method of promoting health in a subject, the method comprising administering to the subject a performance-enhancing formulation and a detoxification formulation, wherein the detoxification formulation is to be administered to the subject within a 24-hour period of administering the performance enhancing formulation to the subject.

## Patentansprüche

1. Orale Formulierung, umfassend:
mehrere Agentien, wobei die mehreren Agentien Brokkolisamenextrakt, Blutorangenextrakt und Traubenkernextrakt beinhalten; und
wobei mindestens eines der mehreren Agentien die Expression von Nrf2-assoziierten Genen bei Ingestion der oralen Formulierung durch eine Person moduliert, wobei die Nrf2-assoziierten Gene mindestens ein Gen, das intrinsische Antioxidantien kodiert, und mindestens ein Gen, das Zell-Entgifter kodiert, beinhalten und wobei mindestens eines der mehreren Agentien eine Entzündung abschwächt.

2. Orale Formulierung nach Anspruch 1, wobei die Nrf2-assoziierten Gene aus der Gruppe bestehend aus NFE2L2, GCLM, GCLC, GSR, GSTA1, GPX1, GPX4, HMOX1, NQO1, SRXN1, SQSTM1, SOD1, UGT1A6, NOS2, NOS3 und PTGS2 ausgewählt sind.

3. Orale Formulierung nach Anspruch 2, wobei mindestens eines der mehreren Agentien eine altersbedingte Veränderung der Expression von mindestens einem der Nrf2-assoziierten Gene im Wesentlichen rückgängig macht.

4. Orale Formulierung nach Anspruch 1, wobei die mehreren Agentien sich kombinieren, um die Expression von mindestens fünf Nrf2-assoziierten Genen zu modulieren.

5. Orale Formulierung nach Anspruch 1, wobei mindestens eines der mehreren Agentien ein Gen, das intrinsische Antioxidantien kodiert, oder ein Gen, das Zell-Entgifter kodiert, hochreguliert oder eine Autophagie in einem Gewebe einer Person stimuliert.

6. Orale Formulierung nach Anspruch 1, wobei die mehreren Agentien zusätzlich mindestens eine bzw. einen bzw. eines von Alpha-Liponsäure, Ganztraubenextrakt, Olivenblattextrakt, Olivenfruchtextrakt, Koenzym Q₁₀, Granatapfelextrakt, Kurkumin, EGCG, Lutein, Lycopin, Zeaxanthin, Resveratrol, Schisandra-Beerenextrakt, Sauerkirsche, Ginseng, Rosmarinextrakt und *Cordyceps sinensis* beinhalten.

7. Orale Formulierung nach Anspruch 1, wobei die Formulierung von 20 bis 30 Gew.-% Brokkolisamenextrakt, von 25 Gew.-% bis 35 Gew.-% Blutorangenextrakt und von 45 bis 55 Gew.-% Traubenkernextrakt umfasst.

8. Orale Formulierung nach Anspruch 1, die in einer Dosisform vorliegt, die aus der Gruppe bestehend aus folgenden ausgewählt ist: Kapsel, Tablette, Pulver, Getränk, Oblate, Konfekt, Kautablette, Gel, Paste, Elixier, Sirup, Tropfen und Pastille.

9. Orale Formulierung nach Anspruch 1, die weiterhin mindestens einen Hilfsstoff umfasst, der aus der Gruppe bestehend aus mikrokristalliner Cellulose, Siliciumdioxid, Stearinsäure, Gummi in Lebensmittelqualität, Lecithin und einem Trennmittel ausgewählt ist.

10. Orale Formulierung nach einem der vorhergehenden Ansprüche zur Verwendung in einer Therapie.

11. Orale Formulierung nach einem der vorhergehenden Ansprüche zur Verwendung bei der Förderung einer therapeutischen Entgiftung in Zellen einer Person.

12. Verwendung der oralen Formulierung nach einem der Ansprüche 1 bis 9 zur Entgiftung in Zellen einer Person, wobei die Verwendung nicht zum Zwecke einer Therapie ist, die an dem menschlichen oder Tierkörper durchgeführt wird.

13. Orale Formulierung zur Verwendung nach Anspruch 10 oder 11, wobei die mehreren Agentien sich kombinieren, um die Expression von mindestens fünf Nrf2-assoziierten Genen zu modulieren.

14. Orale Formulierung zur Verwendung nach Anspruch 10 oder 11, wobei mindestens eines der mehreren Agentien eine Autophagie in einem Gewebe der Person stimuliert.

15. Orale Formulierung zur Verwendung nach Anspruch 10 oder 11, wobei eine Verabreichung die Person mit Folgendem versorgt:
einer Tagesdosis von 125 mg Blutorangenextrakt, 210 mg Traubenkernextrakt und 115 mg Brokkolisamenextrakt oder
einer Dosis von 0,15 mg bis 18 mg Blutorangenextrakt, von 0,3 mg bis 30 mg Traubenkernextrakt und von 0,15 mg bis 16,5 mg Brokkolisamenextrakt pro kg Körpergewicht.

16. Leistungssteigernde Formulierung, die mehrere Agentien umfasst, die die Stoffwechselleistung steigern, und eine wie in einem der Ansprüche 1-9 vorgetragene Entgiftungsformulierung zur Verwendung in einem Verfahren zur Förderung der Gesundheit einer Person, wobei das Verfahren das Verabreichen einer leistungssteigernden Formulierung und einer Entgiftungsformulierung umfasst, wobei die Entgiftungsformulierung an die Person innerhalb eines 24-Stunden-Zeitraums des Verabreichens der leistungssteigernden Formulierung an die Person verabreicht wird.

## Revendications

1. Formulation orale comprenant :
une pluralité d'agents, la pluralité d'agents comprenant un extrait de graines de brocoli, un extrait d'orange sanguine et un extrait de graines de raisin ; et
au moins un agent de la pluralité d'agents modulant une expression de gènes associés à Nrf2 à l'ingestion de la formulation orale par un sujet, les gènes associés à Nrf2 incluant au moins un gène codant des antioxydants intrinsèques, et au moins un gène codant des détoxifiants cellulaires, et au moins un agent de la pluralité d'agents atténuant une inflammation.

2. Formulation orale selon la revendication 1, dans laquelle les gènes associés à Nrf2 sont choisis dans le groupe constitué de NFE2L2, GCLM, GCLC, GSR, GSTA1, GPX1, GPX4, HMOX1, NQ01, SRXN1, SQSTM1, SOD1, UGT1A6, NOS2, NOS3 et PTGS2.

3. Formulation orale selon la revendication 2, dans laquelle au moins un agent de la pluralité d'agents inverse un changement relatif à l'âge dans l'expression d'au moins un des gènes associés à Nrf2.

4. Formulation orale selon la revendication 1, dans laquelle la pluralité d'agents se combinent en une expression de modulation d'au moins cinq gènes associés à Nrf2.

5. Formulation orale selon la revendication 1, dans laquelle au moins un agent de la pluralité d'agents régule positivement un gène codant des antioxydants intrinsèques ou un gène codant des détoxifiants cellulaires, ou stimule une autophagie dans un tissu d'un sujet.

6. Formulation orale selon la revendication 1, dans laquelle la pluralité d'agents comprend en outre au moins un ingrédient choisi parmi de l'acide alpha-lipoïque, un extrait de raisins entiers, un extrait de feuilles d'olivier, un extrait d'olives, de la coenzyme Q10, un extrait de grenade, de la curcumine, de l'EGCG, de la lutéine, du lycopène, de la zéaxanthine, du resvératrol, un extrait de baies de la schizandra, des cerises acides, du ginseng, un extrait de romarin et du *Cordyceps sinensis.*

7. Formulation orale selon la revendication 1, la formulation comprenant de 20 à 30 % en poids d'un extrait de graines de brocoli, de 25 à 35 % en poids d'un extrait d'orange sanguine et de 45 à 55 % en poids d'un extrait de graines de raisin.

8. Formulation orale selon la revendication 1, présente sous forme posologique choisie dans le groupe constitué de : gélule, tablette, poudre, boisson, gaufrette, confiserie, pâte à mâcher, gel, pâte, élixir, sirop, gouttes et pastille.

9. Formulation orale selon la revendication 1, comprenant en outre au moins un excipient choisi dans le groupe constitué de cellulose microcristalline, dioxyde de silicium, d'acide stéarique, d'une gomme alimentaire, de lécithine et d'un agent antimottant.

10. Formulation orale selon l'une quelconque des revendications précédentes, destinée à être utilisée dans une thérapie.

11. Formulation orale selon l'une quelconque des revendications précédentes, destinée à être utilisée pour promouvoir une détoxication thérapeutique dans des cellules d'un sujet.

12. Formulation orale selon l'une quelconque des revendications 1 à 9 en vue d'une détoxication dans des cellules d'un sujet, l'utilisation n'étant pas à des fins d'une thérapie réalisée sur un humain ou un corps animal.

13. Formulation orale destinée à être utilisée selon la revendication 10 ou 11, dans laquelle la pluralité d'agents se combinent en une expression de modulation d'au moins cinq gènes associés à Nrf2.

14. Formulation orale destinée à être utilisée selon la revendication 10 ou 11, dans laquelle au moins un agent de la pluralité d'agents stimule une autophagie dans un tissu du sujet.

15. Formulation orale destinée à être utilisée selon la revendication 10 ou 11, dans laquelle l'administration fournit au sujet :
une dose quotidienne de 125 mg d'extrait d'orange sanguine, 210 mg d'extrait de graines de raisin et 115 mg d'extrait de graines de brocoli ; ou
une dose par kg de poids corporel de 0,15 mg à 18 mg d'extrait d'orange sanguine, de 0,3 mg à 30 mg d'extrait de graines de raisin et de 0,15 mg à 16,5 mg d'extrait de graines de brocoli.

16. Formulation d'amélioration des performances comprenant une pluralité d'agents qui améliorent les performances métaboliques, et formulation de détoxification selon l'une quelconque des revendications 1 à 9, destinées à être utilisées dans un procédé de promotion de la santé chez un sujet, le procédé consistant à administrer au sujet une formulation d'amélioration des performances et une formulation de détoxification, la formulation de détoxification devant être administrée au sujet dans une période de 24 heures suivant l'administration au sujet de la formulation d'amélioration des performances.
